# EUROPEAN PATENT SPECIFICATION

(11) **EP 1 239 811 B1**
(45) Date of publication and mention of the grant of the patent: **25.03.2009**
(21) Application number: 00975631.3
(22) Date of filing: 12.09.2000
(51) Int. Cl.: A61K 8/14, A61K 8/31, A61Q 17/00

(54) **COMPOSITIONS COMPRISING FLUID PETROLATUM**
ZUBEREITUNGEN ENTHALTEND FLÜSSIGES PETROLATUM
COMPOSITIONS DE PETROLATUM FLUIDE

(30) Priority: 15.09.1999 US 396535
(43) Date of publication of application: 18.09.2002
(73) Proprietor: IGEN, INC., Wilmington, DE 19810 (US)
(72) Inventor: MATHUR, Rajiv, Ringoes, NJ 08551 (US); LAWRENCE, Nadya, Cape May, NJ 08204 (US)
(74) Representative: Casey, Lindsay Joseph
(86) International application number: PCT/US2000/040878
(87) International publication number: WO 2001/019326

(56) References cited:
- WO-A-91/04731
- US-A- 4 942 038
- US-A- 5 145 604
- US-A- 5 160 669
- US-A- 5 260 065
- US-A- 5 405 615
- US-A- 5 474 848

## Description

### Background:

Petrolatum and its manufacture were initially patented in 1872 by Robert A. Cheeseborough (U.S. Patent Serial No. 127,568). Although Cheeseborough cited treating leather as its primary use, petrolatum was also recommended as a hair pommenade and for treating chapped hands. In 1875, the American Pharmaceutical Association found petrolatum "without a superior" for treating bums and scalds. Since then, petrolatum's beneficial properties for skin care and treatment have been extensively studied and reported.

Petrolatum has been found to be the best material for relieving ordinary xerosis (Morrison et al.. Cos & Toil. (1996) 111:59). Petrolatum's moisturizing characteristics have been ascribed to the slowed water loss when petrolatum is applied to the skin. Petrolatum has also been used extensively on wound dressings, both as a treatment and as a pharmaceutically acceptable ointment base to deliver other medicinal compositions.

However, the same hydrophobic properties which make petrolatum an effective moisture barrier, also make it difficult to evenly disperse in fluid aqueous preparations. When mixed with water, petrolatum immediately forms a separate distinct layer. Evenly dispersed fluid preparations are essential for commercial manufacturing techniques, such as spraying. This is true for petrolatum encapsulated in lipid vesicles as well as unencapsulated products.

Accordingly, it is an object of the present invention to provide methods and compositions relating to a sprayable petrolatum product consisting of lipid vesicles encapsulating petrolatum, dispersed in an aqueous phase.

Another object of this invention is to provide a stable dispersion of petrolatum containing lipid vesicles in water.

These and other objects and features of the invention will be apparent from the following descriptions.

### Summary of the Invention

The invention is set out in the claims.

### Detailed Description of the Invention

The invention pertains to, at least in part, a sprayable petrolatum product having lipid vesicles encapsulating petrolatum dispersed in an external aqueous phase. The vesicles comprise a primary wall forming material and a metal oxide weighting agent. The primary wall forming material is selected from the group consisting of C₁₂-C₁₈-fatty alcohol, nonionic and zwiterionic surfactants and the weighting agent is present in an amount sufficient that the lipid vesicles have a density of about 0.95-1.0 g/ml. Preferably, petrolatum comprises at least about 20%, e.g., more preferably 30%, by weight of the lipid vesicles. In a further embodiment, the lipid vesicles are paucilamellar.

Petrolatum has been commercially available since the mid-1870's. Commonly, it is a semisolid mixture of hydrocarbons and is, essentially, both odorless and tasteless to humans. Petrolatum is often obtained through the dewaxing of heavy mineral oils.

The primary wall forming material, which constitutes the greatest structural material by weight of the bilayers (e.g., 10-20%), can be any suitable non-ionic surfactant known in the art to be useful in forming vesicles. For example, suitable surfactants are disclosed in US 5,260,065, entitled "Blended Lipid Vesicles;" US 5,234,767, entitled "Lipid Hybrid Vesicles;" US 5,439,967 entitled "Propylene Glycol Stearate Vesicles;" US 5,405,615, entitled "Sucrose Distearate Vesicles," and US 5,160,669, entitled "Method of Making Oil Filled Paucilamellar Lipid Vesicles," the contents all of which are incorporated by reference herein. Advantageously, the primary wall forming material has the property that it will form a lipid vesicle in the absence of said weighting agent. In another embodiment, the primary wall forming material of the vesicle bilayers is selected from the group consisting of polyoxyethylene glyceryl fatty acid esters (e.g., having 1-10 polyoxyethylene groups), such as polyoxyethylene glyceryl monostearate and polyoxyethylene glyceryl monooleate, C₁₂-C₁₈ fatty alcohols, C₁₂-C₁₈ glycol monoesters. C₁₂-C₁₈ glyceryl mono-and diesters, and mixtures thereof. Preferred primary wall forming material are selected from the group consisting of C₁₆ and C₁₈ fatty alcohols, glyceryl mono- and distearate, glyceryl dilaurate, glycol stearate, and mixtures thereof. All of the aforementioned compounds are commercially available. Preferred primary wall forming materials include C₁₆-C₁₈ fatty alcohols, glycol stearate, glyceryl mono- and distearate, glyceryl dilaurate, and combinations thereof.

The term "weighting agent" includes substances which affect the specific gravity of the lipid vesicles. Advantageously, the weighting agents increase the specific gravity of the vesicles to aid dispersion of the vesicles through out an aqueous mixture and to deter separation between the aqueous and lipid components of the mixture. Preferably, the inclusion of the weighting agents increases the specific gravity of the vesicles into the desired stable product range, e.g. greater than 0.95 g/mL to about 0.99 g/mL. In some embodiments of the invention, the weighting agents are incorporated directly into the walls of the vesicles. Preferred weighting agents have a specific gravity greater than 1.0 g/mL, (e.g., greater than 1.0 g/mL to about 2.0 g/mL). Examples include, iron oxide.

The lipid vesicles of the invention may further include one or more charge producing agents which minimize flow of the external aqueous phase into the vesicles. Preferred charge producing agents include negatively charged hydrophilic molecules such as sodium lauryl sulfate, sodium laureth sulfate, sodium lactate, sodium pyrrolidone carboxylate, aloe vera, retinoic acid and urea. Other possible negative charge producing agents include oleic acid, dicetyl phosphate, palmitic acid, cetyl sulphate, retinoic acid, phosphatidic acid, phosphatidyl serine, and mixtures thereof. Alternatively, also contemplated is the incorporation of positively charged molecules in order to provide a net positive charge to the vesicles. Examples of suitable positively charged molecules include, for example, long chain amines, e.g., stearyl amines or oleyl amines, long chain pyridinium compounds, e.g.. cetyl pyridinium chloride, quaternary ammonium compounds, or mixtures of these can be used. A preferred positive charge producing material is hexadecyl trimethylammonium bromide, a potent disinfectant. The use of this disinfectant as the positive charge producing material within the vesicles provides a secondary advantage as the vesicles deteriorate; they act as a sustained release germicide carriers.

The vesicles may also include targeting molecules, either hydrophilic or amphiphilic, which can be used to direct the vesicles to a particular target in order to allow release of the material encapsulated in the vesicle at a specified biological location. If hydrophilic targeting molecules are used, they can be coupled directly or via a spacer to an OH residue of the polyoxyethylene portion of the surfactant, or they can be coupled, using state of the art procedures, to molecules such as palmitic acid, long chain amines, or phosphatidyl ethanolamine. If spacers are used, the targeting molecules can be interdigitated into the hydrophilic core of the bilayer membrane via the acyl chains of these compounds. Preferred hydrophilic targeting molecules include monoclonal antibodies, other immunoglobulins, lectins, and peptide hormones.

In addition to hydrophilic targeting molecules, it is also possible to use amphiphilic targeting molecules. Amphiphilic targeting molecules are normally not chemically coupled to the surfactant molecules but rather interact with the lipophilic or hydrophobic portions of the molecules constituting the bilayer lamellae of the lipid vesicles. Preferred amphiphilic targeting molecules are neutral glycolipids, galactocerebrosides (e.g., for hepatic galactosyl receptors), or charged glycolipids such as gangliosides.

The vesicles of the invention may further comprise sterols. Sterols useful in forming the lipid bilayers also include any sterol known in the art to be useful as modulators of lipid membranes. Suitable sterols include but are not limited to cholesterol, cholesterol derivatives, hydrocortisone, phytosterol, or mixtures thereof, In one embodiment, the sterol is phytosterol supplied from avocado oil unsaponifiables. The use of this sterol, in particular, to form lipid vesicles is described in issued U.S-Patent No. 5,643,600, entitled "Lipid Vesicles Containing Avocado Oil Unsaponifiables".

In another embodiment, the invention pertains to a method for protecting the skin of a mammal. The method involves contacting the skin with a sprayable pharmaceutical petrolatum composition product which contains lipid vesicles encapsulating petrolatum dispersed in an external aqueous phase. The lipid vesicles are comprised of a primary wall forming material and a weighting agent. The primary wall forming material is a nonionic or a zwiterionic surfactant. The weighting agent is present in an amount sufficient such that the lipid vesicles have a density of about 0.95-1.0 g/ml.

Petrolatum filled vesicles may be formed using either the "hot loading" technique disclosed in U. S. Patent No. 4,911,928, entitled "Paucilamellar Lipid Vesicles." or the "cold loading" technique described in U. S. Patent No. 5,160,669, entitled, "Method of Making Oil Filled Paucilamellar Lipid Vesicles." In either case, a lipid phase is formed by blending a primary wall forming material and the weighting agent, along with any other materials to be incorporated into the lipid bilayers, to form a homogenous lipid phase. In the "hot loading" technique, the petrolatum is blended in the already formed lipid phase, forming a lipophilic phase.

Once a lipophilic phase is made, it is blended with an aqueous phase (e.g., water, saline, or any other aqueous solution which will be used to hydrate the lipids) under shear mixing conditions to form the vesicles. "Shear mixing conditions", as used herein, means a shear equivalent to a relative flow of 5-50 m/s through a 1 mm orifice. The paucilamellar lipid vesicles of the disclosure can be made by a variety of devices which provides sufficiently high shear for shear mixing. A device which is particularly useful for making the lipid vesicles of the present invention is described in U. S. Patent No. 4,895,452, entitled "Method and apparatus for producing lipid vesicles."

In the "cold loading" technique, the lipid phase and the aqueous phase are blended under shear mixing conditions to form vesicles. Once the substantially aqueous filled lipid vesicles are formed, they are combined with the "cargo" material to be encapsulated, e.g., the petrolatum. Droplets of the water immiscible material enter the vesicles, presumably by a process resembling endocytosis. The vesicles are subsequently blended under low shear conditions, as described in U. S. Patent No. 5,160,669.

The invention is further illustrated by the following Examples.

### Exemplification of the Invention:

### REFERENCE EXAMPLE 1: Petrolatum Filled Vesicles

The following procedure was used to make vesicles of the invention. Tables 1-3 describe three different formulations of vesicles of the invention.

The lipid components of each vesicle preparation were weighed out into a stainless steel kettle and heated to 80°C. The materials were mixed together until a clear solution was obtained. Each clear solution was then cooled to 73- 75 °C.

The aqueous component of each vesicle preparations were weighed into a separate stainless steel kettle. An additional 2% of water was added to compensate for evaporation. Each solution was heated to 58-60 °C.

Using a Novamix^{™} lipid vesicle machine (described in United States Patent No. 4,895,452), the lipid and aqueous components of each vesicle preparation were separately mixed in a 1:1.70 ratio. Each sample was then stirred continuously and allowed to cool to room temperature before being stored. Microscopic analysis revealed small, regular, spherical vesicles for each of the sample formulations.

The vesicles of each sample were transferred directly from storage to a spraying apparatus, without being further diluted. Each of the samples was sprayed onto a black and white surface and analyzed. All of the samples were found to be satisfactory and commercially viable.

**Table 1**

| | **Ingredients** | % | **Kg** (for 100 Kg prep.) |
|---|---|---|---|
| **LIPID PHASE** | Glyceryl Distearate (Kessco. Stepan) | 3.60 | 3.60 |
| | Steareth-10 (Brij 76, ICI) | 2.00 | 2.00 |
| | Cholesterol USP (Maypro) | 1.00 | 1.00 |
| | Polysorbate 80 (Protameen) | 0.50 | 0.50 |
| | White Petrolatum USP (Penreco) | 30.00 | 30.00 |
| **AQUEOUS PHASE** | DI Water | 60.15 | 60.15 |
| | Urea USP | 2.00 | 2.00 |
| | Germaben II (ISP Van Dyk)* | 0.75 | 0.75 |

**Table 2**

| | **Ingredients** | % | **Kg** (for 100 Kg prep.) |
|---|---|---|---|
| **LIPID PHASE** | Glyceryl Distearate (Kessco. Stepan) | 3.60 | 3.60 |
| | Steareth-10 (Brij 76, ICI) | 2.00 | 2.00 |
| | Cholesterol USP (Maypro) | 1.00 | 1.00 |
| | Polysorbate 80 (Protameen) | 0.50 | 0.50 |
| | White Petrolatum USP (Penreco) | 30.00 | 30.00 |
| **AQUEOUS PHASE** | DI Water | 63.95 | 63.95 |
| | Sodium Laureth Sulfate (30%) | 1.00 | 1.00 |
| | Germaben II-E^{*} | 0.75 | 0.75 |

| | | | |
|---|---|---|---|
| * propylene glycol, diazolidinyl urea, methyl paraben and propyl paraben. | | | |

**Table 3**

| | **Ingredients** | % | **Kg** (for 1500 Kg.) |
|---|---|---|---|
| **LIPID PHASE** | Glyceryl Distearate (Kessco. Stepan) | 3.60 | 54.00 |
| | Steareth-10 (Brij 76, ICI) | 2.00 | 30.00 |
| | Cholesterol USP (Maypro) | 1.00 | 15.00 |
| | Polysorbate 80 (Protameen) | 0.50 | 7.50 |
| | White Petrolatum USP (Penreco) | 30.00 | 450.00 |
| **AQUEOUS PHASE** | DI Water | 60.58 | 908.70 |
| | Sipon LSB (30% SLS) | 2.00 | 30.00 |
| | Methyl Paraben | 0.20 | 3.00 |
| | Propyl Paraben | 0.03 | 0.45 |
| | Sodium Chloride | 0.09 | 1.35 |

### EQUIVALENTS

Those skilled in the art will recognize, or be able to ascertain using no more than routine experimentation, many equivalents to the specific embodiments and methods described herein. Such equivalents are intended to be encompassed by the scope of the claims.

## Claims

1. A sprayable petrolatum composition having lipid vesicles encapsulating petrolatum dispersed in an external aqueous phase, said lipid vesicles comprising a primary wall forming material, and a weighting agent, said primary wall forming material being selected from the group consisting of C₁₂-C₁₈ fatty alcohols, and nonionic and zwiterionic surfactants, and said weighting agent being present in an amount sufficient that said lipid vesicles have a density of 0.95-1.0 g/ml, **characterised in that** the weighting agent is a metal oxide.

2. A composition as claimed in claim 1, wherein said primary wall forming material has the property that it will form a lipid vesicle in the absence of said weighting agent.

3. A composition as claimed in claim 1 or claim 2, wherein said primary wall forming material is selected from the group consisting of polyoxyethylene glyceryl fatty acids, C₁₂-C₁₈ glycol monoesters, C₁₂-C₁₈ glyceryl mono-and diesters, polyoxyethylene fatty alcohols, betaines, sarcosinates, propylene glycerol stearate, sucrose distearate, glycerol bilaurate, glucosides, and mixtures thereof.

4. A composition as claimed in any preceding claim wherein said lipid vesicle is a paucilamellar lipid vesicle.

5. A composition as claimed in any preceding claim wherein said weighting agent is incorporated into the wall of said lipid vesicle.

6. A composition as claimed in any preceding claim, wherein said weighting agent has a specific gravity greater than 1.0 g/ml.

7. A composition as claimed in any preceding claim, wherein said weighting agent has a specific gravity between 1.0 g/ml and 2.0 g/ml.

8. A composition as claimed in any preceding claim, wherein said lipid vesicles further comprise a charge producing agent which minimizes flow of said external aqueous phase into said vesicles.

9. A composition as claimed in claim 8, wherein said charge producing agent is selected from the group consisting of sodium lauryl sulfate, sodium laureth sulfate, sodium lactate, sodium pyrrolidone carboxylate, retinoic acid, aloe vera and urea.

10. A composition as claimed in claim 1, wherein said petrolatum comprises at least 20% by weight of said lipid vesicles.

11. A composition as claimed in claim 1, wherein said petrolatum comprises at least 30% by weight of said lipid vesicle.

## Patentansprüche

1. Sprühbare Petrolat-Zusammensetzung mit Lipidvesikeln, die das in einer externen wässrigen Phase dispergierte Petrolat einkapseln, wobei die Lipidvesikel ein primäres Wandbildnermaterial und ein Beschwerungsmittel umfassen, wobei das primäre Wandbildnermaterial aus der Gruppe ausgewählt ist, die aus C₁₂-C₁₈-Fettalkoholen, und nicht ionischen und zwitterionischen Tensiden besteht, und wobei das Beschwerungsmittel in einer ausreichenden Menge vorliegt, dass die Lipidvesikel eine Dichte von 0,95-1,0 g/ml aufweisen, **dadurch gekennzeichnet, dass** das Beschwerungsmittel ein Metalloxid darstellt.

2. Zusammensetzung nach Anspruch 1, worin das primäre Wandbildnermaterial die Eigenschaft aufweist, dass es in der Abwesenheit des Beschwerungsmittels ein Lipidvesikel bildet.

3. Zusammensetzung nach Anspruch 1 oder 2, worin das primäre Wandbildnermaterial aus der Gruppe ausgewählt ist, die aus Folgendem besteht: Polyoxyethylen-Glycerylfettsäuren, C₁₂-C₁₈-Glykolmonoestern, C₁₂-C₁₈-Glycerylmono- und -diestern, Polyoxyethylen-Fettalkoholen, Betainen, Sarcosinaten, Propylenglycerolstearat, Saccharose-Distearat, Glyceroldilaurat, Glucoside und Gemischen davon.

4. Zusammensetzung nach einem der vorangehenden Ansprüche, worin das Lipidvesikel ein paucilamellares Lipidvesikel darstellt.

5. Zusammensetzung nach einem der vorangehenden Ansprüche, worin das Beschwerungsmittel in die Wand des Lipidvesikels inkorporiert ist.

6. Zusammensetzung nach einem der vorangehenden Ansprüche, worin das Beschwerungsmittel eine relative Dichte von größer als 1,0 g/ml aufweist.

7. Zusammensetzung nach einem der vorangehenden Ansprüche, worin das Beschwerungsmittel eine relative Dichte zwischen 1,0 g/ml und 2,0 g/ml aufweist.

8. Zusammensetzung nach einem der vorangehenden Ansprüche, worin die Lipidvesikel ferner ein ladungsproduzierendes Mittel umfassen, das den Fluss der externen wässrigen Phase in die Vesikel minimiert.

9. Zusammensetzung nach Anspruch 8, worin das ladungsproduzierende Mittel aus der Gruppe ausgewählt ist, die aus Natriumlaurylsulfat, Natriumlaurethsulfat, Natriumlaktat, Natriumpyrrolidoncarboxylat, Retinsäure, Aloe vera und Harnstoff besteht.

10. Zusammensetzung nach Anspruch 1, worin das Petrolat mindestens 20 Gew.-% der Lipidvesikel umfasst.

11. Zusammensetzung nach Anspruch 1, worin das Petrolat mindestens 30 Gew.-% des Lipidvesikels umfasst.

## Revendications

1. Composition de pétrolatum pulvérisable ayant des vésicules lipidiques encapsulant du pétrolatum dispersées dans une phase aqueuse externe, lesdites vésicules lipidiques comprenant un matériau de formation de paroi primaire, et un agent de charge, ledit matériau de formation de paroi primaire étant choisi parmi le groupe constitué par les alcools gras en C₁₂-C₁₈, et les agents tensioactifs non ioniques et zwitterioniques, et ledit agent de charge étant présent en une quantité suffisante pour que lesdites vésicules lipidiques aient une densité de 0,95-1,0 g/ml, **caractérisée en ce que** l'agent de charge est un oxyde métallique.

2. Composition selon la revendication 1, dans laquelle ledit matériau de formation de paroi primaire possède la propriété selon laquelle il forme une vésicule lipidique en absence dudit agent de charge.

3. Composition selon la revendication 1 ou la revendication 2, dans laquelle ledit matériau de formation de paroi primaire est choisi parmi le groupe constitué par les acides gras de glycéryle polyoxyéthylénés, les monoesters de glycol en C₁₂-C₁₈, les mono- et di-esters de glycéryle en C₁₂-C₁₈, les alcools gras polyoxyéthylénés, les bétaïnes, les sarcosinates, le stéarate de propylène glycérol, le distéarate de saccharose, le bilaurate de glycérol, les glucosides, et des mélanges de ceux-ci.

4. Composition selon l'une quelconque des revendications précédentes, dans laquelle ladite vésicule lipidique est une vésicule lipidique paucilamellaire.

5. Composition selon l'une quelconque des revendications précédentes, dans laquelle ledit agent de charge est incorporé dans la paroi de ladite vésicule lipidique.

6. Composition selon l'une quelconque des revendications précédentes, dans laquelle ledit agent de charge a une gravité spécifique supérieure à 1,0 g/ml.

7. Composition selon l'une quelconque des revendications précédentes, dans laquelle ledit agent de charge a une gravité spécifique comprise entre 1,0 g/ml et 2,0 g/ml.

8. Composition selon l'une quelconque des revendications précédentes, dans laquelle lesdites vésicules lipidiques comprennent en outre un agent producteur de charge qui minimise l'écoulement de ladite phase aqueuse externe dans lesdites vésicules.

9. Composition selon la revendication 8, dans laquelle ledit agent producteur de charge est choisi parmi le groupe constitué par le laurylsulfate de sodium, le sodium laureth sulfate, le lactate de sodium, le pyrrolidonecarboxylate de sodium, l'acide rétinoïque, l'aloé vera et l'urée.

10. Composition selon la revendication 1, dans laquelle ledit pétrolatum comprend au moins 20% en poids desdites vésicules lipidiques.

11. Composition selon la revendication 1, dans laquelle ledit pétrolatum comprend au moins 30% en poids de ladite vésicule lipidique.
